(19) Europäisches Patentamt
European Patent Office
Office européen des brevets

(11) Publication number: 0 153 016 B1

(12) EUROPEAN PATENT SPECIFICATION

(45) Date of publication of patent specification: 16.05.90

(51) Int. Cl.$^5$: C 07 D 211/90, C 07 D 413/14, A 61 K 31/44

(21) Application number: 85300384.6

(22) Date of filing: 21.01.85

# (54) Asymmetrical diesters of 1,4-dihydro-2,6-dimethyl-pyridine-3,5-dicarboxylic acid.

(30) Priority: 14.02.84 GB 8403866

(43) Date of publication of application: 28.08.85 Bulletin 85/35

(45) Publication of the grant of the patent: 16.05.90 Bulletin 90/20

(84) Designated Contracting States: AT BE CH DE FR GB IT LI LU NL SE

(56) References cited:
EP-A-0 060 897
EP-A-0 071 819
EP-A-0 094 159
EP-A-0 128 010

(73) Proprietor: RECORDATI S.A. CHEMICAL and PHARMACEUTICAL COMPANY
Corso S. Gottardo 54
CH-6830 Chiasso (CH)

(72) Inventor: Nardi, Dante
Via T. Gulli 47
Milan (IT)
Inventor: Leonardi, Amedeo
Via Poliziano 16
Milan (IT)
Inventor: Graziani, Gabriele
Via dei Platini 8
Arese Milan (IT)
Inventor: Bianchi, Giorgio
Via Vallazze 100
Milan (IT)

(74) Representative: SERJEANTS
25, The Crescent King Street
Leicester, LE1 6RX (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 153 016 B1

Courier Press, Leamington Spa, England.

**Description**

The invention relates to asymmetrical diesters of 1,4-dihydro-2,6-dimethyl-pyridine-3,5-dicarboxylic acid, to stereochemically isomeric forms and pharmaceutically acceptable acid addition salts thereof, to processes for their production and to pharmaceutical compositions containing them.

Nicardipine is a known compound having antihypertensive properties. It is methyl N-benzyl-N-methyl-2-aminoethyl 1,4-dihydro-2,6-dimethyl-4-(3-nitrophenyl)-pyridine-3,5-dicarboxylate. Various attempts have been made to find a more effective antihypertensive compound based on this structure. It is known to replace the 4-(3-nitrophenyl) group with another aryl group and to replace the methyl esterifying group with another alkyl or alkoxyalkyl group. Various proposals have been made for replacing the N-benzyl-N-methyl-2-aminoethyl group.

EP—A—0060897 discloses the use of an N-(aralkyl or alkyl)-N-(aralkyl or alkyl)-1-aryl-(2-aminoethyl or 3-aminopropyl) group. The most favoured such groups appear to be B-benzyl-N-methyl-1-(phenyl or substituted phenyl)-2-aminoethyl groups. There is no specific disclosure of an N(aralkyl or alkyl)-N-(aralkyl or alkyl) substitution pattern other than N-benzyl-N-methyl, and only one specific disclosure of a 3-aminopropyl group being used rather than a 2-aminoethyl group. The aminoalkyl group, whether 2-aminoethyl or 3-aminopropyl, is essentially of straight chain configuration.

EP—A—0094159 discloses the replacement of the 2-aminoethyl group by a piperazinyl-alkyl or homopiperazinyl-alkyl group. In this there is only one nitrogen atom available for substitution, so the N-benzyl-N-methyl terminus is replaced by an N-aryl or N-aralkyl terminus. Diphenylmethyl is one of the preferred N-aralkyl termini. It is said that the alkylene group in the piperazinyl-alkyl or homopiperazinyl-alkyl preferably has 2 to 4 carbon atoms and may be straight chained or branched chained, this being exemplified by ethylene, trimethylene, propylene, tetramethylene and 1,2-dimethylethylene (page 3 lines 19 to 22). However, ethylene is most clearly preferred, being incorporated in all but two of more than sixty specifically disclosed compounds. The other two (Examples 37 and 38) incorporate the straight chained trimethylene.

Our invention, like those of EP—A—0060897 and EP—A—0094159, is based on replacing the N-benzyl-N-methyl-2-aminoethyl group of nicardipine. In particular, we have replaced the aminoethyl group by an aminoalkyl group of branched chain configuration, and the N-benzyl group with a 3-3-diphenylpropyl or 1-methyl-3,3-diphenylpropyl group, neither of which has been specifically disclosed previously.

Accordingly the invention provides asymmetrical diesters of 1,4-dihydro-2,6-dimethyl-pyridine-3,5-dicarboxylic acid, the esters having the general formula

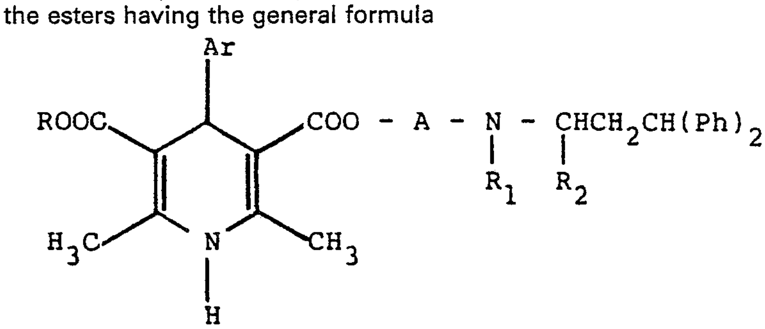

(I)

wherein

Ph represents a phenyl group,

Ar represents a 2-nitrophenyl, 3-nitrophenyl, 2,3-dichlorophenyl or benzofurazan-4-yl group,

A represents a branched chain alkylene group containing from 2 to 6 carbon atoms,

R represents a straight chain or branched chain alkyl group having from 1 to 6 carbon atoms, optionally mono-substituted by an alkoxy group having from 1 to 6 carbon atoms,

$R_1$ represents a hydrogen atom, a hydroxy group or an alkyl group having from 1 to 4 carbon atoms, and

$R_2$ represents a hydrogen atom or a methyl group; and further provides pharmaceutically acceptable acid addition salts of such diesters.

The invention further provides a process for the preparation of the esters of the general formula I, the process comprising condensing a compound of the general formula II

$$Ar—CHO \qquad (II)$$

wherein Ar is as above defined with a compound of the general formula III

$$CH_3COCH_2COOA_1 \qquad (III)$$

wherein $A_1$ represents either (a) a group of the general formula IV

$$—A—N(R_1)—CH(R_2)—CH_2—CH(Ph)_2 \qquad (IV)$$

wherein A, $R_1$, $R_2$ and Ph are as above defined or (b) a group of the general formula AX wherein A is as above defined and X represents a halogen atom, reacting the condensate with a compound of the general formula V

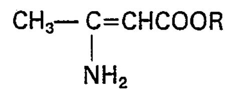

(V)

wherein R is as above defined to give a compound of the general formula (VI)

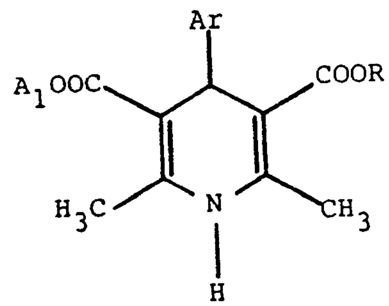

(VI)

wherein $A_1$, R and Ar are as above defined, and if $A_1$ represents the group AX as above defined then converting it to a group of the general formula IV as above defined.

It will be understood that various synthetic routes are encompassed within the above process. The reaction scheme below illustrates some of these.

For example, the esters I may be prepared by condensing a haloalkyl acetoacetate IIIb (III: $A_1$ = AX) with an aldehyde II, reacting the condensate with an alkyl or alkoxyalkyl 3-aminocrotonate V, and converting the group AX of the resultant 1,4-dihydropyridine derivative VIb (VI: $A_1$ = AX) to a group IV by reaction with 3,3-diphenyl-propylamine or a derivative thereof (VII). Alternatively, the group IV may be introduced into the compound III before ring formation. This route starts from compound VIII

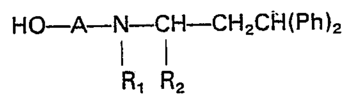

VIII

wherein A, $R_1$, $R_2$ and Ph are as above defined. This is available from the amine VII by conventional alkylation to introduce a hydroxyalkyl group HO—A and reaction of the alkylated amine with diketene. Compound IIIa (III: $A_1$=IV) is condensed with an aldehyde II and the product is reacted with a 3-aminocrotonate V.

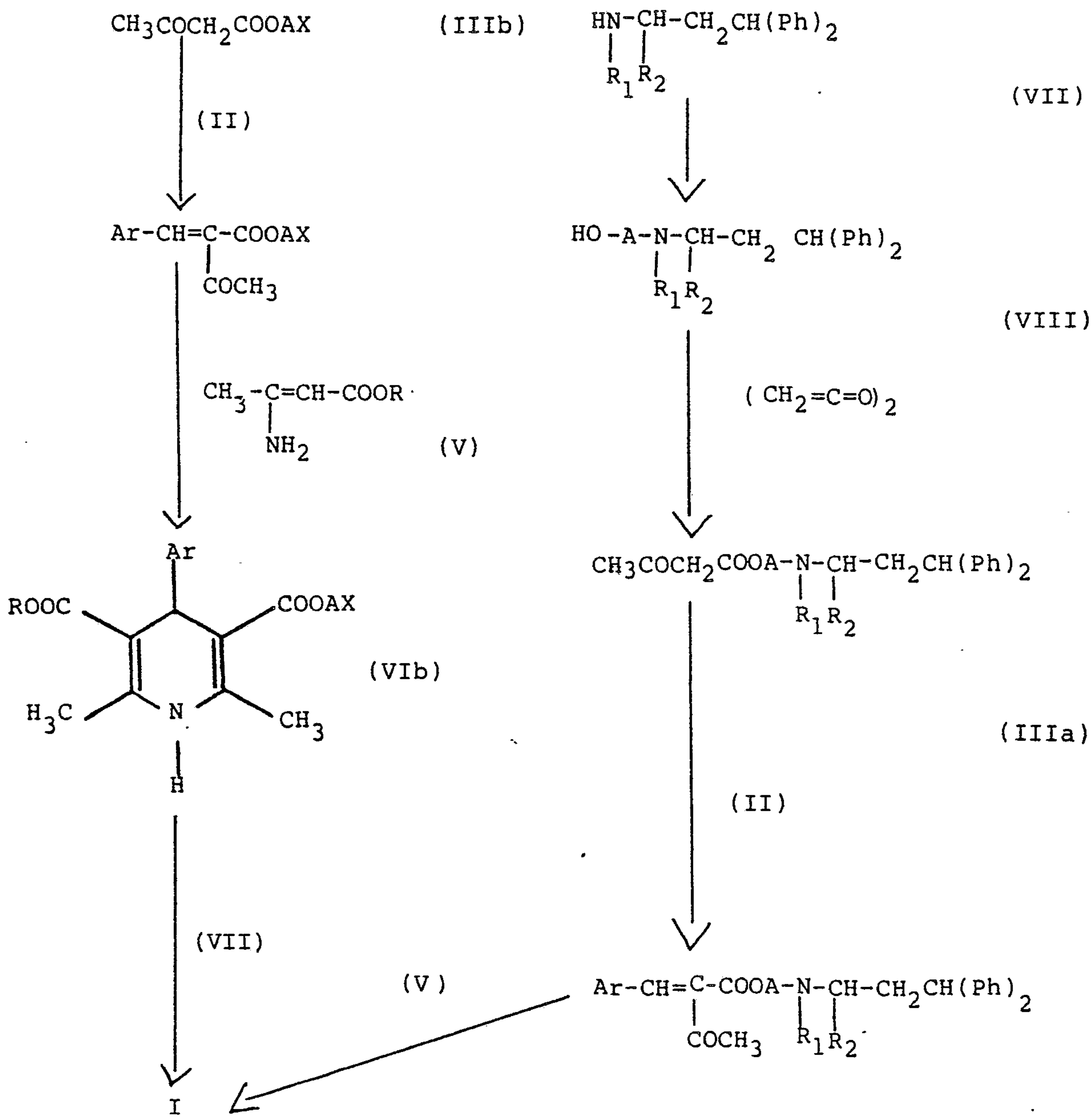


The above process includes a synthesis of the pyridine ring. If a 1,4-dihydropyridine derivative VIb is already available it is only necessary to condense it with an amine VII. Similarly if a free acid of formula IX

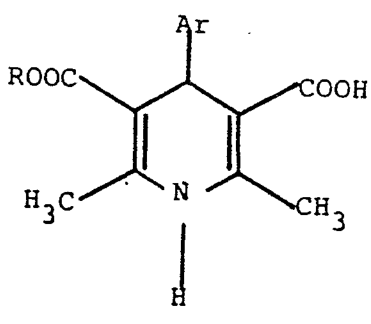

(IX)

wherein R and Ar are as above defined is available it is only necessary to condense it with a compound VIII, or with a compound of the general formula YAX wherein Y represents a halogen atom and A and X are as above defined to give a 1,4-dihydropyridine derivative VIb for condensation with an amine VII. These condensations are themselves within the scope of the invention. When X represents a chlorine atom, they are preferably carried out in toluene or xylene under reflux, whereas when X represents a bromine atoms they may be carried out in dimethylformamide at lower temperature.

The diesters I obtained may be purified according to methods known per se, and crystallized as salts from suitable solvents in purified form. The pharmaceutically acceptable salts according to the invention may be prepared from the bases in a conventional manner. Preferred pharmaceutically acceptable acid addition salts are those of hydrochloric, sulphuric, maleic, succinic, citric, methanesulphonic and toluenesulphonic acids.

The diesters I and their salts according to the invention possess a valuable antihypertensive activity and are also effective against coronary heart diseases. Accordingly, the invention also provides a pharmaceutical composition comprising a diester of the general formula I as above defined or a pharmaceutically acceptable salt thereof in admixture with a pharmaceutically acceptable diluent or carrier.

The $LD_{50}$ of the compounds according to the invention was determined in the mouse both i.p. and per os, following the method described by C.S. Weil (Biometrics, 8, 249, 1952).

The antihypertensive activity of the esters according to the invention was evaluated in male hypertensive rats (SHR, Wister-Kyoto strain, 15—25 weeks old). The determination of blood pressure was performed by an indirect method (M. Gerald et al., Arzneim. Forsch., 18,1285, 1968). The animals were prewarmed in a heating chamber at a temperature of from 35° to 37°C for a period of 15 minutes before pressure determination. The compounds tested by oral route were dissolved or suspended in a 0.5% methylcellulose solution. Controls were given only the vehicle. Systolic blood pressure and heart rate were measured 1,3,5, and 7 hours after drug administration by means of a tail-cuff and a pulse transducer.

Coronary dilating activity were evaluated in anesthetized normotensive rats (weighing about 500 g), as the ability to antagonize methacholine induced coronary spasm. Rats were instrumented for methacholine infusion into the coronary ostium, while spastic activity was detected as ST segment elevation in $D_2$ ECG recording (K. Sakai et al., J. Pharm. Meth., *5,* 325, 1981).

The compounds tested by i.v. infusion were dissolved in water: dimethylformamide (9:1 by volume). Activity was detected as normalization of ECG tracing after compounds administration during methachholine infusion. The former test indicates that the esters possess valuable antihypertensive activities; according to the methacholine test the compounds according to the invention can also be considered effective against coronary heart diseases.

| Compound | $LD_{50}$ i.p. | mg/kg os | $ED_{25}$SHR os mg/kg | $ED_{50}$ iv ug/kg |
|---|---|---|---|---|
| 2288 | 72 | 197 | 1.5 | 46 |
| 2375 | 83 | 657 | 2.6 | 82 |
| 2383 | — | 500 | 3.4 | — |

$ED_{25}$ = antihypertensive activity
$ED_{50}$ = coronary dilating activity
— = not tested

The following Examples illustrate the invention.

Example 1

1-Methyl-2-chloroethyl α-acetyl-3-nitro-cinnamate

A solution containing 12.2 g of 3-nitrobenzaldehyde and 14.3 g of 1-methyl-2-chloroethyl acetoacetate in 80 ml of toluene, maintained at 0.5°C, was saturated with hydrogen chloride gas. After two days at 20—25°C, the residual hydrogen chloride was removed by bubbling nitrogen through the solution. The toluene was then evaporated off in vacuo. The residual oil was dissolved in dichloromethane and the solution thus obtained was washed with water until it was neutral. The organic phase which was separated and dried, was then evaporated to dryness under vacuum at 20°C. The residue was crystallized from 200 ml of isopropanol to give 20.70 g of the title compound, melting at 95—96°C.

Operating as described above, but employing 2,3-dichlorobenzaldehyde, the 1-methyl-2-chloroethyl α-acetyl-2,3-dichloro-cinnamate was obtained as an oil.

These compounds are mixtures of E,Z isomers and were employed as such for further reactions without separating the components.

Example 2

Methyl 1-methyl-2-chloroethyl 1,4-dihydro-2,6-dimethyl-4-(3-nitrophenyl)-pyridine-3,5-dicarboxylate

A solution comprising 18.72 g of 1-methyl-2-chloroethyl α-acetyl-3-nitro-cinnamate, prepared as described in Example 1, and 7.12 g of methyl 3-amino-crotonate in 48 ml of isopropanol was refluxed under stirring for 210 minutes. After cooling, the reaction mixture was evaporated to dryness under vacuum and the oil thus obtained was chromatographed on silica gel (200 g) using chloroform as eluent. Evaporating the fractions containing a sole product (TLC, chloroform:ethylacetate 95:5) gave a thick oil, which was dissolved in diethyl ether. The solvent was then evaporated off at 20—25°C to give 15.75 g of the title compound, m.p. 95—102°C, which could be employed without further purification.

Operating as described above, but using isobutyl 3-aminocrotonate and 1-methyl-2-chloromethyl α-acetyl-2,3-dichlorocinnamate (obtained as reported in Example 1), the intermediate isobutyl 1-methyl-2-chloroethyl 1,4-dihydro-2,6-dimethyl-4-(2,3-dichlorophenyl)-pyridine-3,5-dicarboxylate was obtained as a low melting point solid. It was purified by column chromatography or by crystallization from a suitable solvent.

Example 3

Methyl 1-N-dimethyl-N-(3,3-diphenylpropyl)-2-aminoethyl 1,4-dihydro-2,6-dimethyl-4-(3-nitrophenyl)-pyridine-3,5-dicarboxylate (2288)

A solution comprising 6.13 of methyl 1-methyl-2-chloroethyl 1,4-dihydro-2,6-dimethyl-4-(3-nitrophenyl)-pyridine,3,5-dicarboxylate and 10.13 g of N-methyl-3,3-diphenylpropylamine in 20 ml of xylene was refluxed under stirring for 105 minutes. The mixtures was then cooled. By dilution with diethyl ether a semisolid compound was obtained, and it was separated from the liquid by decantation. The residue was treated with diethyl ether at 0.4°C and the supernatant was decanted. This procedure was repeated until a solid was obtained, and this was collected by filtration. Mother liquors and washings from the decantation were collected and evaporated to dryness under vacuum. The residue was chromatographed on a silica gel column (270 g) using a mixture of chloroform and acetone as eluent. Pure fractions were combined and the solvent was evaporated off. The residue was dissolved in diethyl ether and hydrogen chloride in diethyl ether was added. The solid was crystallized from ethyl acetate or isopropyl acetate and recrystallized from ethyl acetate to give the title compound, m.p. (as hydrochloride) 114—123°C.

Example 4

Methyl 1-N-dimethyl-N-(3,3-diphenylpropyl)-2-aminopropyl 1,4-dihydro-2,6-dimethyl-4-(3-nitrophenyl)-pyridine-3,5-dicarboxylate (2366)

A solution of 3.72 of 3-oxo-but-1-ene was added dropwise, over a period of 20 minutes and so that the temperature did not exceed 2—3°C, to a cooled solution of 7.87 g of N-methyl-3,3-diphenylpropylamine in 7 ml of diethyl ether. The solution thus obtained was evaporated in vacuo at room temperature to give 10.3 g of N-methyl-N-(3,3-diphenylpropyl)-4-amino-butan-2-one (100%) as free base, shown to be practically pure by TLC (chloroform:methanol 9:1 by volume) which could be used as such for further reactions. This compound may be transformed into the corresponding hydrochloride (m.p. 133—135°C) by treating the free base with hydrogen chloride in diethyl ether, and crystallizing the product thus obtained first from acetone:ethyl acetate:isopropanol then from acetone.

To a solution of 9.16 g of the compound obtained as described above in 30 ml of methanol and maintained at 0°C, within 5 minutes 0.90 g of sodium borohydride were slowly added. When the addition was over, the methanol was evaporated off in vacuo and the residue treated with water (about 100 ml) and diethyl ether (about 100 ml). The water was then washed with diethyl ether and the ethereal phases were combined, dried on anhydrous sodium sulphate and evaporated to dryness in vacuo. The product (9.25 g) thus obtained, N-methyl-N-(3,3-diphenyl-propyl)-4-aminobutan-2-ol, was a reddish oil unitary at TLC (chloroform:methanol 9:1 by volume).

A suspension comprising 7.44 g of the product from the last step, 12.46 g of 1,4-dihydro-2,6-dimethyl-4-(3-nitrophenyl)-5-methoxycarbonylpyridine-3-carboxylic acid, 8.51 g of N,N'-dicyclohexylcarbodiimide and 0.54 g of 4-dimethylaminopyridine in 50 ml of dimethylformamide was stirred at room temperature for 8 days. The suspension was then diluted with 450 ml of diethyl ether and the N,N'-dicyclohexylurea thus precipitated was collected by filtration. The filtered solution was washed with water (3 × 200 ml). The organic phase was dried and then evaporated off in vacuo. The oil thus obtained was purified by flash chromatography on silica gel columns using chloroform:ethyl acetate mixtures (1:1 by volume) as eluent. The fractions which were unitary at TLC (ethyl acetate) were evaporated to give a semi-solid residue. This residue was dissolved in diethyl ether and the solution thus obtained was filtered and acidified with hydrogen chloride in diethyl ether to give a solid which was collected by filtration. The solution of this solid in 200 ml of ethyl acetate was diluted with 600 ml of ether, maintaining acetate:diethyl ether ratio of 1:3 by volume. The mixture was stirred at 0°C until a precipitate was obtained. The operation was repeated three times, to give 8.50 g of the hydrochloride of the title compound, m.p. 92—110°C.

Example 5

2, N-dimethyl-N-(3,3-diphenylpropyl)-1-amino-2-propanol

A mixture of 16.87 g of N-methyl-3,3-diphenylpropylamine and 3.1 ml of 1-chloro-2-methyl-2-propanol

in 20 ml of xylene was refluxed under stirring for 8 hours. After cooling at 15—20°C, the suspension was diluted with diethyl ether and the starting amine hydrochloride thus obtained was collected by filtration. The solvent was removed from the filtrate in vacuo to give an oil which was then purified by flash chromatography on silica gel columns, employing chloroform with increasing amounts of methanol as eluent. The unitary TLC fractions(chloroform:methanol 94.6 by volume) were evaporated to give 7.8 g of the title compound as a brown oil.

Example 6

N-Methyl-N-(3,3-diphenylpropyl)-1-amino-2-propanol

6.38 g of 1,2-epoxypropane was added to a solution of 22.5 g of N-methyl-N-(3,3-diphenylpropylamine in 60 ml of methanol. The solution was maintained at 15—20°C for 3 days, and then a further 1.16 g of 1,2-epoxypropane was added. This solution was maintained for 24 hours at room temperature. The methanol was evaporated off and the product purified by chromatography on a silica gel column (methanol gradient in chloroform), to give 24 g of the title compound as colourless oil.

Example 7

1,1,N-Trimethyl-N-(3,3-diphenylpropyl)-2-aminoethyl acetoacetate

A solution of 13.10 g of the compound in prepared in Example 5 in 10 ml of toluene was heated to 85°C. 3.6 ml of diketene were added dropwise over a period of 10 minutes maintaining the temperature below 100°C. The solution was then heated for 2 hours at 80°C. After cooling at 15—20°C the mixture was evaporated in vacuo and the oily residue was purified by flash chromatography on silica gel columns using chloroform containing increasing amounts of methanol as eluent. The unitary TLC fractions (chloroform:methanol 95:5 by volume) were evaporated to dryness to give 12.3 g of the title compound as a brown oil.

Example 8

1,N-Dimethyl-N-(3,3-diphenylpropyl)-2-aminoethyl acetoacetate

Operating as described in Example 7, but using the compound prepared in Example 6 instead of that prepared in Example 5, the title compound was obtained as an oil.

Example 9

1,1,N-Trimethyl-N-(3,3-diphenylpropyl)-2-aminoethyl α-acetyl-3-nitro-cinnamate

Hydrogen chloride was bubbled into a solution of 3.78 g of 3-nitrobenzaldehyde and 9.54 g of the compound prepared in Example 7 in 25 ml of chloroform, cooled at 0°C, until the solution was saturated. The solution was stood for 3 days at 15°C. It was then diluted with chloroform and washed with dilute aqueous sodium hydroxide solution until neutrality. The organic phase was dried and the solvent was evaporated off. The solid thus obtained was dissolved in ethyl acetate and the solution was cooled at 0°C. A slight excess of hydrogen chloride in diethyl ether was added. The solid thus formed was repeatedly treated with diethyl ether to give 12.50 g of the title compound, m.p. 65—80°C. The product was an E/Z isomeric mixture, and was used as such in futher reactions.

Example 10

1,N-Dimethyl-N-(3,3-diphenylpropyl)-2-aminoethyl α-acetyl-3-nitrocinnamate

Operating as described in Example 9, but using the compound prepared in Example 8 instead of that prepared in Example 7, the title compound was obtained as an oil. The product was an E/Z isomeric mixture, and was used as such in further reactions.

Example 11

1,N-Dimethyl-N-(3,3-diphenylpropyl)-2-aminoethyl α-acetyl-2,3-dichlorocinnamate

Operating as described in Example 10, but using 2,3-dichlorobenzaldehyde instead of 3-nitrobenzaldehyde, the title compound was obtained as an oil. The product was an E/Z isomeric mixture, and was used as such in further reactions.

Example 12

Methyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate (2375)

A solution of 2.37 g of methyl 3-aminocrotonate and 10.29 g of the compound prepared in Example 9 in 15 ml of isopropanol was refluxed for 3 hours. The mixture was then cooled and evaporated to dryness in vacuo. The oily residue thus obtained was purified by flash chromatography on silica gel columns, using chloroform with increasing amounts of acetone as eluent. Te unitary TLC fractions (chloroform:acetone, 9:1 by volume) were evaporated and the solid thus obtained was dissolved in methanol. A slight excess of ethanolic hydrogen chloride was added and the solution was evaporated to dryness. The residue was dissolved in acetone and again evaporated in vacuo. The residue was crystallized from 2 litres of water containing 2 ml of 1N hydrochloric acid and 5 ml of water saturated with sodium chloride, to give 4.8 g of the hydrochloride hemihydrate of the title compound, m.p. 119—123°C.

Example 13

Isobutyl 1,N-dimethyl-N-(3,3-diphenylpropyl)-2-aminoethyl 1,4-dihydro-2,6-dimethyl-4-(2,3-dichlorophenyl)-pyridine-3,5-dicarboxylate (2383)

Operating as described in Example 12, but using isobutyl 3-aminocrotonate instead of methyl 3-aminocrotonate and the compound of Example 11 instead of that of Example 9, the hydrochloride of the title compound, m.p. 162—164°C, was obtained.

Example 14

2-Propoxyethyl 1,N-dimethyl-N-(3,3-diphenylpropyl)-2-aminoethyl 1,4-dihydro-2,6-dimethyl-4-(2,3-dichlorophenyl)-pyridine-3,5-dicarboxylate (2400)

Operating as described in Example 13, but using 2-propoxyethyl 3-aminocrotonate instead of isobutyl 3-aminocrotonate the hydrochloride hydrate of the title compound, m.p. 108—118°C, was obtained.

Example 15

2-Propoxyethyl 1,N-dimethyl-N-(3,3-diphenylpropyl)-2-aminoethyl 1,4-dihydro-2,6-dimethyl-4-(3-nitrophenyl)-pyridine-3,5-dicarboxylate (2408)

Operating as described in Example 14, but using the compound of Example 10 instead of that of Example 11, the hydrochloride hemihydrate of the title compound, m.p. 85—92°C, was obtained.

Example 16

2-Propoxyethyl 1,1-dimethylethyl 1-N-dimethyl-N-(3,3-diphenylpropyl)-2-aminoethyl 1,4-dihydro-2,6-dimethyl-4-(3-nitrophenyl)-pyridine-3,5-dicarboxylate (2409)

Operating as described in Example 15, but using 2-propoxy-1,1-dimethylethyl 3-aminocrotonate instead of 2-propoxyethyl 3-aminocrotonate, the hydrochloride hemihydrate of the title compound, m.p. 86—95°C, was obtained.

Example 17

2-Propoxyethyl 1,1-trimethyl-N-(3,3-diphenylpropyl)-2-aminoethyl 1,4-dihydro-2,6-dimethyl-4-(3-nitrophenyl)-pyridine-3,5-dicarboxylate (2411)

Operating as described in Example 12, but using 2-propoxyethyl 3-aminocrotonate instead of methyl 3-aminocrotonate, the hydrorochloride hemihydrate of the title compound, m.p. 87—92°C, was obtained.

Example 18

2-propoxy-1,1-dimethyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate (2412)

Operating as described in Example 12, but using 2-propoxy-1,1-dimethylethyl -aminocrotonate instead of methyl 3-aminocrotonate, the hydrochloride hemihydrate of the title compound, m.p. 88—92°C, was obtained.

**Claims for the Contracting States: BE CH LI DE FR GB IT LU NL SE**

1. An asymmetrical diester of 1,4-dihydro-2,6-dimethyl-pyridine-3,5-dicarboxylic acid, the ester having the general formula I

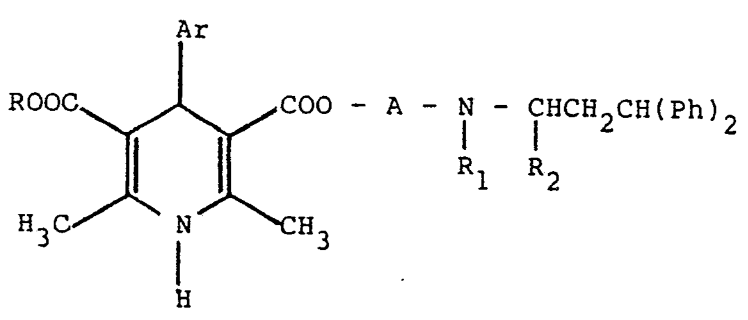

(I)

wherein

Ph represents a phenyl group,

Ar represents a 2-nitrophenyl, 3-nitrophenyl, 2,3-dichlorophenyl or benzofurazan-4-yl group,

A represents a branched chain alkylene group containing from 2 to 6 carbon atoms,

R represents a straight chain or branched chain alkyl group having from 1 to 6 carbon atoms, optionally mono-substituted by an alkoxy group having from 1 to 6 carbon atoms,

$R_1$ represents a hydrogen atom, a hydroxy group or an alkyl group having from 1 to 4 carbon atoms, and

$R_2$ represents a hydrogen atom or a methyl group;

a stereochemically isomeric form thereof or a pharmaceutically acceptable acid addition salt thereof.

2. Any of the following compounds or their pharmaceutically acceptable acid addition salts:

Methyl 1,N-dimethyl-N-(3,3-diphenylpropyl)-2-aminoethyl 1,4-dihydro-2,6-dimethyl-4-(3-nitrophenyl)-pyridine-3,5-dicarboxylate,

Methyl 1,N-dimethyl-N-(3,3-diphenylpropyl)-3-aminopropyl 1,4-dihydro-2,6-dimethyl-4-(3-nitrophenyl)-pyridine-3,5-dicarboxylate,

Methyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate,

Isobutyl 1,N-dimethyl-N-(3,3-diphenylpropyl)-aminoethyl 1,4-dihydro-2,6-dimethyl-4-(2,3-dichlorophenyl)-pyridine-3,5-dicarboxylate,

2-Propoxyethyl 1,N-dimethyl-N-(3,3-diphenylpropyl)-2-aminoethyl 1,4-dihydro-2,6-dimethyl-4-(2,3-dichloronyl)-pyridine-3,5-dicarboxylate,

2-Propoxyethyl 1,N-dimethyl-N-(3,3-diphenylpropyl)-2-aminoethyl 1,4-dihydro-2,6-dimethyl-4-(3-nitrophenyl)-pyridine-3,5-dicarboxylate,

2-Propoxy-1,1-dimethylethyl 1,N-dimethyl-N-(3,3-diphenylpropyl)-2-aminoethyl 1,4-dihydro-2,6-dimethyl-4-(3-nitrophenyl)-pyridine-3,5-dicarboxylate,

2-Propoxyethyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate, and

2-Propoxy-1,1-dimethylethyl 1,1,N-trimethyl-N-(3,3-diphenylpropyl)-2-aminoethyl 1,4-dihydro-2,6-dimethyl-4-(3-nitrophenyl)-pyridine-3,5-dicarboxylate.

3. A process for the preparation of a diester according to claim 1, the process comprising condensing a compound of the general formula II

$$Ar—CHO \qquad (II)$$

wherein Ar is as defined in claim 1 with a compound of the general formula III

$$CH_3COCH_2COOA_1 \qquad (III)$$

wherein $A_1$ represents (a) a group of the general formula IV

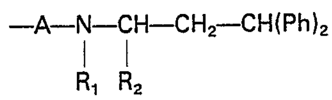

(IV)

wherein A, $R_1$, $R_2$ and Ph are as defined in claim 1 or (b) a group of the general formula AX wherein A is as defined in claim 1 and X represents a halogen atom, reacting the condensate with a compound of the general formula V

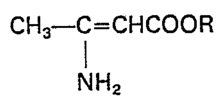

wherein R is as defined in claim 1 to give a compound of the general formula VI

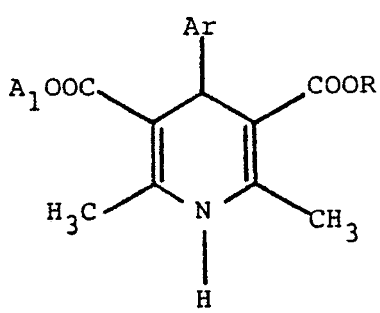

(VI)

wherein $A_1$ is as defined in this claim and Ar and R are as defined in claim 1, and if $A_1$ represents a group of the general formula AX then converting it to a group of the general formula IV.

4. A process according to claim 3 in which $A_1$ represents a group of the formula AX and the conversion of the group AX to a group of the general formula IV is effected by reacting the compound of the general formula VI with a 3,3-diphenyl-propylamine derivative of the general formula VII

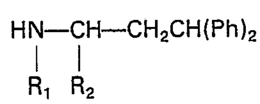

wherein $R_1$, $R_2$ and Ph are as defined in claim 1.

5. A process for the preparation of a diester according to claim 1, the process comprising condensing a pyridine derivative of the general formula

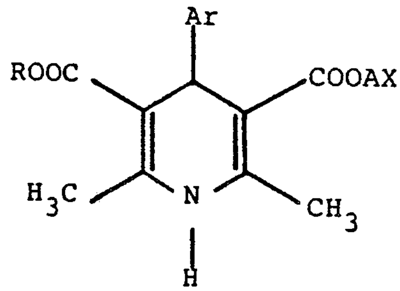

wherein Ar, R and A are as defined in claim 1 and X represents a halogen atom with a 3,3-diphenylpropylamine derivative of the general formula VII as defined in claim 4.

6. A process for the preparation of a diester according to claim 1, the process comprising condensing a pyridine derivative of the general formula

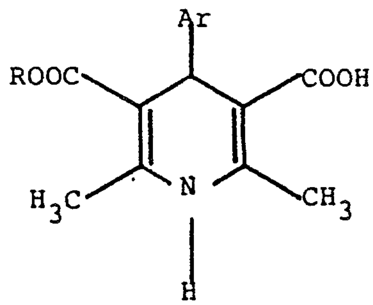

wherein R and Ar are as defined in claim 1 with a compound of the general formula VIII

$$HO{-}A{-}\underset{R_1}{N}{-}\underset{R_2}{CH}{-}CH_2CH(Ph)_2 \qquad (VIII)$$

wherein A, $R_1$, $R_2$ and Ph are as defined in claim 1.

7. A process for the preparation of a diester according to claim 1, the process comprising condensing a pyridine derivative of the general formula

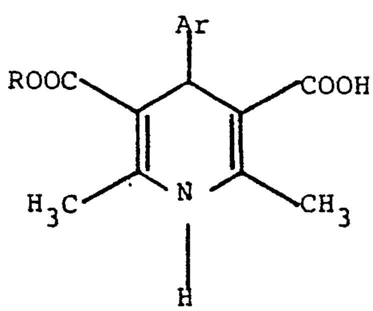

wherein R and Ar are as defined in claim 1 with a compound of the general formula YAX wherein A is as defined in claim 1 and X and Y represent halogen atoms, and proceeding according to claim 5.

8. A pharmaceutical composition comprising a diester according to claim 1 or claim 2 or a pharmaceutically acceptable acid addition salt thereof in admixture with a pharmaceutically acceptable diluent or carrier.

**Claims for the Contracting State: AT**

1. A process for the preparation of an asymmetrical diester of 1,4-dihydro-2,6-dimethyl-pyridine-3,5-dicarboxylic acid, the ester having the general formula I

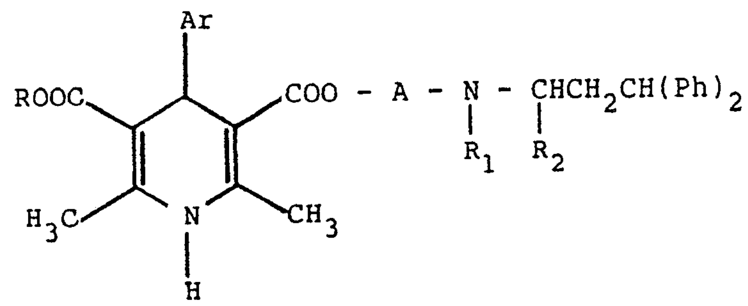

(I)

wherein

Ph represents a phenyl group,

Ar represents a 2-nitrophenyl, 3-nitrophenyl, 2,3-dichlorophenyl or benzofurazan-4-yl group,

A represents a branched chain alkylene group containing from 2 to 6 carbon atoms,

R represents a straight chain or branched chain alkyl group having from 1 to 6 carbon atoms, optionally mono-substituted by an alkoxy group having from 1 to 6 carbon atoms,

$R_1$ represents a hydrogen atom, a hydroxy group or an alkyl group having from 1 to 4 carbon atoms, and

$R_2$ represents a hydrogen atom or a methyl group;

the process comprising condensing a compound of the general formula II

$$Ar-CHO \qquad (II)$$

wherein Ar is as defined in this claim 1 with a compound of the general formula III

$$CH_3COCH_2COOA_1 \qquad (III)$$

wherein $A_1$ represents (a) a group of the general formula IV

$$-A-\underset{R_1}{\underset{|}{N}}-\underset{R_2}{\underset{|}{CH}}-CH_2-CH(Ph)_2 \qquad (IV)$$

wherein A, $R_1$, $R_2$ and Ph are as defined in this claim or (b) a group of the general formula AX wherein A is as defined in this claim and X represents a halogen atom, reacting the condensate with a compound of the general formula V

$$CH_3-\underset{NH_2}{\underset{|}{C}}=CHCOOR \qquad (V)$$

wherein R is as defined in this claim 1 to give a compound of the general formula VI

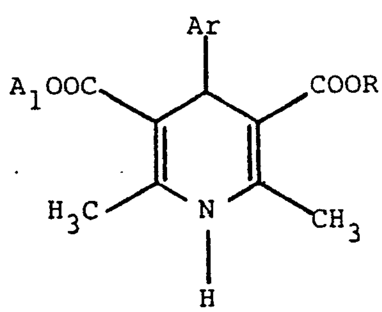

(VI)

wherein $A_1$, Ar and R are as defined in this claim, and if $A_1$ represents a group of the general formula AX then converting it to a group of the general formula IV.

2. A process according to claim 1 in which $A_1$ represents a group of the formula AX and the conversion of the group AX to a group of the general formula IV is effected by reacting the compound of the general formula VI with a 3,3-diphenyl-propylamine derivative of the general formula VII

$HN—CH—CH_2CH(Ph)_2$
| |
$R_1$ $R_2$

wherein $R_1$, $R_2$ and Ph are as defined in claim 1.

3. A process for the preparation of a diester as defined in claim 1, the process comprising condensing a pyridine derivative of the general formula

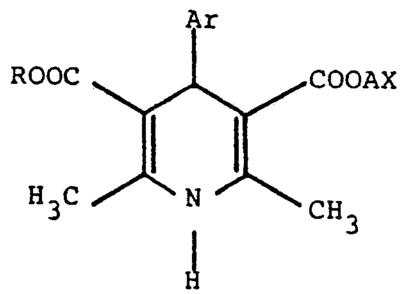

wherein Ar, R, A and X are as defined in claim 1 with a 3,3-diphenyl-propylamine derivative of the general formula VII as defined in claim 2.

4. A process for the preparation of a diester as defined in claim 1, the process comprising condensing a pyridine derivative of the general formula

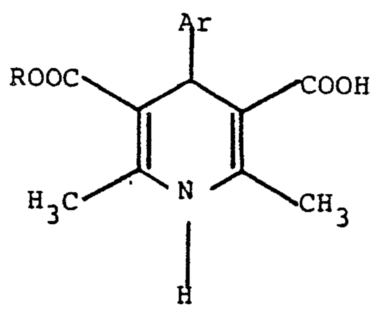

wherein R and Ar are as defined in claim 1 with a compound of the general formula VIII

$HO—A—N—CH—CH_2CH(Ph)_2$ (VIII)
| |
$R_1$ $R_2$

wherein A, $R_1$, $R_2$ and Ph are as defined in claim 1.

5. A process for the preparation of a diester as defined in claim 1, the process comprising condensing a pyridine derivative of the general formula

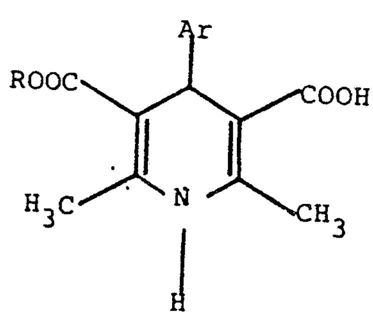

wherein R and Ar are as defined in claim 1 with a compound of the general formula YAX wherein A is as defined in claim 1 and X and Y represent halogen atoms, and proceeding according to claim 3.

**Patentansprüche für die Vertragsstaaten: BE CH LI DE FR GB IT LU NL SE**

1. Ein asymmetrischer Diester von 1,4-Dihydro-2,6-dimethyl-pyridin-3,5-Dicarbonsäure, welcher der allgemeinen Formel I entspricht

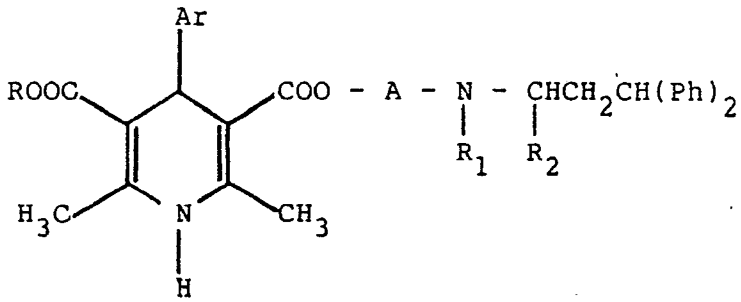

(I)

in welcher Ph eine Phenylgruppe bedeutet, Ar eine 2-Nitrophenyl-, 3-Nitrophenyl-, 2,3-Dichlorphenyl- oder Bezofurazan-4-yl-Gruppe bedeutet, A eine verzweigtkettige Alkylengruppe mit 2 bis 6 C-Atomen bedeutet, R eine geradkettige oder verzweigte Alkylgruppe mit 1 bis 6 C-Atomen, welche gegebenenfalls durch eine Alkoxygruppe mit 1 bis 6 C-Atomen monosubstituiert ist, bedeutet, $R_1$ Wasserstoff, eine Hydroxylgruppe oder eine Alkylgruppe mit 1 bis 4 C-Atomen bedeutet und $R_2$ Wasserstoff oder eine Methylgruppe bedeutet, eine stereochemisch isomere Form oder ein pharmazeutisch anwendbares Säureadditionssalz davon.

2. Irgendeine der folgenden Verbindungen oder ihre pharmazeutisch anwendbaren Säureadditionssalz:

Methyl 1,N-dimethyl-N-(3,3-diphenylpropyl)-2-aminoäthyl 1,4-dihydro-2,6-dimethyl-4-(3-nitrophenyl)-pyridin-3,5-dicarboxylat;

Methyl 1,N-dimethyl-N-(3,3-diphenylpropyl)-3-aminopropyl 1,4-dihydro-2,6-dimethyl-4-(3-nitrophenyl)-pyridin-3,5-dicarboxylat;

Methyl 1,1,N-trimethyl-N-(3,3-diphenylpropyl)-2-aminoäthyl 1,4-dihydro-2,6-dimethyl-4-(3-nitrophenyl)-pyridin-3,5-dicarboxylat;

Isobutyl 1,N-dimethyl-N-(3,3-diphenylpropyl)-2-aminoäthyl 1,4-dihydro-2,6-dimethyl-4-(2,3-dichlorphenyl)-pyridin-3,5-dicarboxylat;

2-Propoxyäthyl 1,N-dimethyl-N-(3,3-diphenylpropyl)-2-aminoäthyl 1,4-dihydro-2,6-dimethyl-4-(2,3-dichlorphenyl)-pyridin-3,5-dicarboxylat;

2-Propoxyäthyl 1,N-dimethyl-N-(3,3-diphenylpropyl)-2-aminoäthyl 1,4-dihydro-2,6-dimethyl-4-(3-nitrophenyl)-pyridin-3,5-dicarboxylat;

2-Propoxy-1,1-dimethyläthyl 1,N-dimethyl-N-(3,3-diphenylpropyl)-2-aminoäthyl 1,4-dihydro-2,6-dimethyl-4-(3-nitrophenyl)-pyridin-3,5-dicarboxylat;

2-Propoxyäthyl 1,1,N-trimethyl-N-(3,3-diphenylpropyl)-2-aminoäthyl 1,4-dihydro-2,6-dimethyl-4-(3-nitrophenyl)-pyridin-3,5-dicarboxylat; und

2-Propoxy-1,1-dimethyläthyl 1,1,N-trimethyl-N-(3,3-diphenylpropyl)-2-aminoäthyl 1,4-dihydro-2,6-dimethyl-4-(3-nitrophenyl)-pyridin-3,5-dicarboxylat.

3. Verfahren zur Herstellung eines Diesters nach Anspruch 1, dadurch gekennzeichnet, dass eine Verbindung der allgemeinen Formel II

$$Ar—CHO \qquad (II)$$

worin Ar wie in Anspruch 1 definiert ist mit einer Verbindung der allgemeinen Formel III

$$CH_3COCH_2COOA_1 \qquad (III)$$

worin $A_1$ (a) eine Gruppe der allgemeinen Formel IV

$$—A—\underset{R_1}{\underset{|}{N}}—\underset{R_2}{\underset{|}{CH}}—CH_2—CH(Ph)_2 \qquad (IV)$$

in welcher A, $R_1$, $R_2$ und Ph wie in Anspruch 1 definiert sind, oder (b) eine Gruppe der allgemeinen Formel AX, worin A wie in Anspruch 1 definiert ist, und X ein Halogenatom bedeutet, kondensiert wird, das Kondensat mit einer Verbindung der allgemeinen Formel V

$$CH_3—\underset{NH_2}{\underset{|}{C}}=CHCOOR \qquad (V)$$

in welcher R wie in Anspruch 1 definiert ist, zur Reaktion gebracht wird, um eine Verbindung der allgemeinen Formel VI zu ergeben

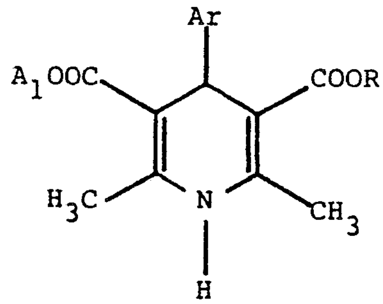

(VI)

in welcher $A_1$ wie in diesem Anspruch definiert ist und Ar und R wie in Anspruch 1 definiert sind und im Falle, dass $A_1$ eine Gruppe der allgemeinen Formel AX darstellt diese in eine Gruppe der allgemeinen Formel IV umgewandelt wird.

4. Verfahren nach Anspruch 3, wobei $A_1$ eine Gruppe der Formel AX bedeutet und die Umwandlung der Gruppe AX in eine Gruppe der allgemeinen Formel IV durch Reaktion der Verbindung der allgemeinen Formel VI mit einem 3,3-Diphenylpropylamin-Derivat der allgemeinen Formel VII

$$\underset{R_1}{\underset{|}{HN}}—\underset{R_2}{\underset{|}{CH}}—CH_2CH(Ph)_2 \qquad (VII)$$

worin $R_1$, $R_2$ und Ph wie in Anspruch 1 definiert sind, ausgeführt wird.

5. Verfahren zur Herstellung eines Diesters nach Anspruch 1, dadurch gekennzeichnet, dass ein Pyridinderivat der allgemeinen Formel

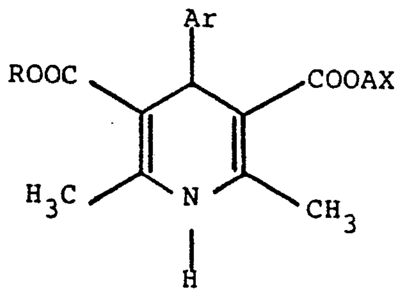

in welcher Ar, R und A wie in Anspruch 1 definiert sind und X ein Halogenatom bedeutet, mit einem 3,3-Diphenylpropylamin-Derivat der allgemeinen Formel VII, wie in Anspruch 4 definiert, kondensiert wird.

6. Verfahren zur Herstellung eines Diesters nach Anspruch 1, dadurch gekennzeichnet, dass ein Pyridinderivat der allgemeinen Formel

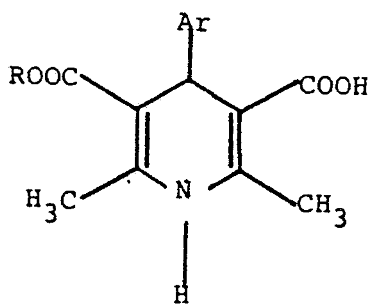

in welcher R und Ar wie in Anspruch 1 definiert sind, mit einer Verbindung der allgemeinen Formel VIII

$$HO—A—\underset{R_1}{\underset{|}{N}}—\underset{R_2}{\underset{|}{CH}}—CH_2CH(Ph)_2 \qquad (VIII)$$

in welcher A, $R_1$, $R_2$ und Ph wie in Anspruch 1 definiert sind, kondensiert wird.

7. Verfahren zur Herstellung eines Diesters nach Anspruch 1, dadurch gekennzeichnet, dass ein

Pyridinderivat der allgemeinen Formel

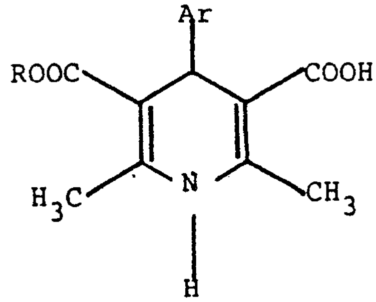

in welcher R und Ar wie in Anspruch 1 definiert sind, mit einer Verbindung der allgemeinen Formel YAX, worin A wie in Anspruch 1 definiert ist und X und Y Halogenatome darstellen, kondensiert wird und entsprechend Anspruch 5 vorgegangen wird.

8. Pharmazeutische Zubereitung bestehend aus einem Diester nach Anspruch 1 oder Anspruch 2 oder einem pharmazeutisch anwendbaren Säureadditionssalz davon in Mischung mit einem pharmazeutisch anwendbaren Verdünnungsmittel oder Trägerstoff.

**Patentansprüche für den Vertragsstaat: AT**

1. Verfahren zur Herstellung eines asymmetrischen Diesters von 1,4-Dihydro-2,6-dimethyl-pyridin-3,5-Dicarbonsäure, welcher der allgemeinen Formel I entspricht

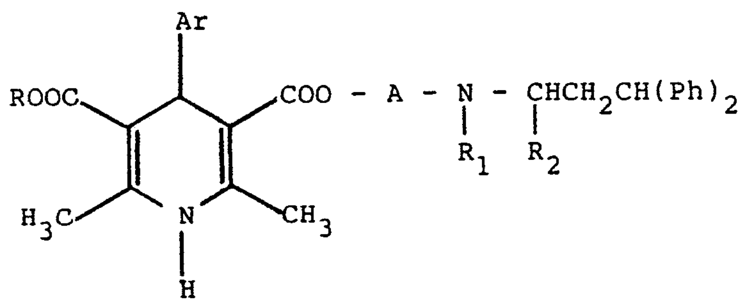

(I)

in welcher Ph eine Phenylgruppe bedeutet, Ar eine 2-Nitrophenyl-, 3-Nitrophenyl-, 2,3-Dichlorphenyl- oder Bezofurazan-4-yl-Gruppe bedeutet, A eine verzweigtkettige Alkylengruppe mit 2 bis 6 C-Atomen bedeutet, R eine geradkettige oder verzweigte Alkylgruppe mit 1 bis 6 C-Atomen, welche gegebenenfalls durch eine Alkoxygruppe mit 1 bis 6 C-Atomen monosubstituiert ist, bedeutet, $R_1$ Wasserstoff, eine Hydroxylgruppe oder eine Alkylgruppe mit 1 bis 4 C-Atomen bedeutet und $R_2$ Wasserstoff oder eine Methylgruppe bedeutet, dadurch gekennzeichnet, dass eine Verbindung der allgemeinen Formel II

$$Ar—CHO \qquad (II)$$

worin Ar wie in diesem Anspruch definiert ist, mit einer Verbindung der allgemeinen Formel III

$$CH_3COCH_2COOA_1 \qquad (III)$$

in welcher $A_1$ (a) eine Gruppe der allgemeinen Formel IV

$$—A—\underset{R_1}{\underset{|}{N}}—\underset{R_2}{\underset{|}{CH}}—CH_2—CH(Ph)_2 \qquad (IV)$$

worin A, $R_1$, $R_2$ und Ph wie in diesem Anspruch definiert sind, oder (b) eine Gruppe der allgemeinen Formel AX, in welcher A wie in diesem Anspruch definiert ist und X ein Halogenatom darstellt, kondensiert wird, das Kondensat mit einer Verbindung der allgemeinen Formel V

$$—CH_3—\underset{NH_2}{\underset{|}{C}}=CHCOOR \qquad (V)$$

in welcher R wie in diesem Anspruch definiert ist, zur Reaktion gebracht wird, um eine Verbindung der allgemeinen Formel VI

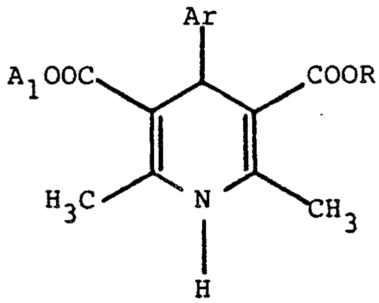

(VI)

worin $A_1$, Ar und R wie in diesem Anspruch definiert sind, zu ergeben, und im Fall, dass $A_1$ eine Gruppe der allgemeinen Formel AX bedeutet, diese in eine Gruppe der allgemeinen Formel IV umgewandelt wird.

2. Verfahren nach Anspruch 1, wobei $A_1$ eine Gruppe der Formel AX bedeutet und die Umwandlung der Gruppe AX in eine Gruppe der allgemeinen Formel IV durch Reaktion der Verbindung der allgemeinen Formel VI mit einem 3,3-Diphenylpropylamin-Derivat der allgemeinen Formel VII

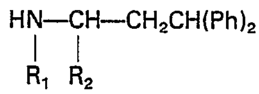

(VII)

worin $R_1$, $R_2$ und Ph wie in Anspruch 1 definiert sind, ausgeführt wird.

3. Verfahren zur Herstellung eines Diesters nach Anspruch 1, dadurch gekennzeichnet, dass ein Pyridinderivat der allgemeinen Formel

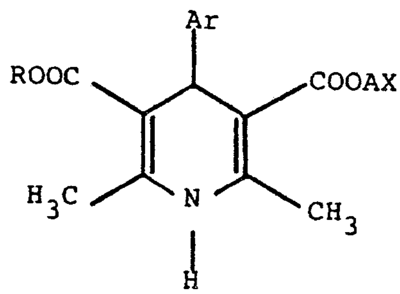

in welcher Ar, R, A und X wie in Anspruch 1 definiert sind, mit einem 3,3-Diphenylpropylamin-Derivat der allgemeinen Formel VII wie in Anspruch 2 definiert, kondensiert wird.

4. Verfahren zur Herstellung eines Diesters nach Anspruch 1, dadurch gekennzeichnet, dass ein Pyridinderivat der allgemeinen Formel

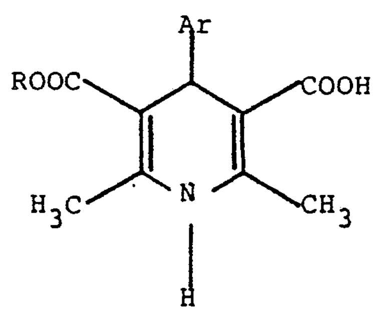

in welcher R und Ar wie in Anspruch 1 definiert sind, mit einer Verbindung der allgemeinen Formel VIII

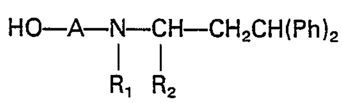

(VIII)

worin A, $R_1$, $R_2$ und Ph wie in Anspruch 1 definiert sind, kondensiert wird.

5. Verfahren zur Herstellung eines Diesters nach Anspruch 1, dadurch gekennzeichnet, dass ein Pyridinderivat der allgemeinen Formel

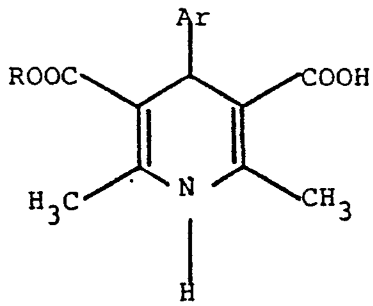

in welcher R und Ar wie in Anspruch 1 definiert sind, mit einer Verbindung der allgemeinen Formel YAX, worin A wie in Anspruch 1 definiert ist und X und Y Halogenatome bedeutet, kondensiert wird und entsprechend Anspruch 3 vorgegangen wird.

**Revendications pour les Etats contractants: BE CH LI DE FR GB IT LU NL SE**

1. Un diester asymétrique de l'acide dihydro-1,4 diméthyl-2,6 pyridine dicarboxylique-3,5, l'ester répondant à la formule générale I

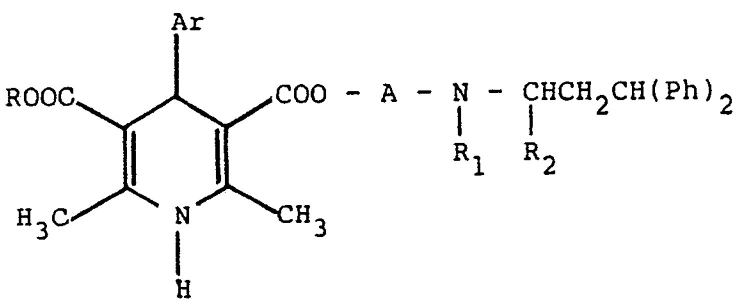

(I)

dans laquelle

Ph représente un groupement phényl,

Ar représente un groupement nitro-2 phényl, nitro-3 phényl, dichloro-2,3 phényl ou benzofurazane 4-yl,

A représente un groupement alcoylène en chaîne ramifiée comportant de 2 à 6 atomes de carbone,

R représente un groupement alcoyle en chaîne droite ou ramifiée ayant de 1 à 6 atomes de carbone, éventuellement monosubstitué par un groupement alcoxy ayant de 1 à 6 atomes de carbone,

$R_1$ représente un atome d'hydrogène, un groupement hydroxy ou un groupement alcoyle ayant de 1 à 4 atomes de carbone, et

$R_2$ représente un atome d'hydrogène ou un groupement méthyl;

une forme stéréochimiquement isomère ou un sel d'addition acide pharmaceutiquement acceptable de celui-ci.

2. L'un quelconque des composés suivants ou leurs sels d'addition acide pharmaceutiquement acceptables:

Méthyl diméthyl-1,N N-(diphényl-3,3 propyl) amino-2 éthyl dihydro-1,4 diméthyl-2,6 (nitro-3 phényl)-4 pyridine dicarboxylate-3,5,

Méthyl diméthyl-1,N N-(diphényl-3,3 propyl) amino-3 propyl dihydro-1,4 diméthyl-2,6 (nitro-3 phényl)-4 pyridine dicarboxylate-3,5,

Méthyl triméthyl-1,1,N N-(diphényl-3,3 propyl) amino-2 éthyl dihydro-1,4 diméthyl-2,6 (nitro-3 phényl)-4 pyridine dicarboxylate-3,5,

Isobutyl diméthyl-1,N N-(diphényl-3,3 propyl) amino-2 éthyl dihydro-1,4 diméthyl-2,6 (dichloro-2,3 phényl)-4 pyridine dicarboxylate-3,5,

Propoxyéthyl-2 diméthyl-1,N N-(diphényl-3,3 propyl) amino-2 éthyl dihydro-1,4 diméthyl-2,6 (dichloro-2,3 phényl)-4 pyridine dicarboxylate-3,5,

Propoxyéthyl-2 diméthyl-1,N N-(diphényl-3,3 propyl) amino-2 éthyl dihydro-1,4 diméthyl-2,6 (nitro-3 phényl)-4 pyridine dicarboxylate-3,5,

Propoxy-2 diméthyl-1,1 éthyl diméthyl-1,N N-(diphényl-3,3 propyl) amino-2 éthyl dihydro-1,4 diméthyl-2,6 (nitro-3 phényl)-4 pyridine dicarboxylate-3,5,

Propoxyéthyl-2 triméthyl-1,1,N N-(diphényl-3,3 propyl) amino-2 éthyl, dihydro-1,4 diméthyl-2,6 (nitro-3 phényl)-4 pyridine dicarboxylate-3,5, et

Propoxy-2 diméthyl-1,1 éthyl triméthyl-1,1,N N-(diphényl-3,3 propyl) amino-2 éthyl dihydro-1,4 diméthyl-2,6 (nitro-3 phényl)-4 pyridine dicarboxylate-3,5.

3. Un procédé pour la préparation d'un diester selon la revendication 1, ledit procédé consistant à condenser un composé de formule générale II

$$Ar{-}CHO \qquad (II)$$

dans laquelle Ar est tel que défini dans la revendication 1, sur un composé de formule générale III

$$CH_3COCH_2COOA_1 \qquad (III)$$

dans laquelle $A_1$ représente (a) un groupement de formule générale IV

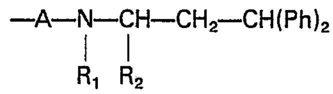

(IV)

dans laquelle A, $R_1$, $R_2$ et Ph sont tels que définis dans la revendication 1 ou (b) un groupement de formule générale AX dans laquelle A est tel que défini dans la revendication 1 et X représente un atome d'halogène, à faire réagir le produit de la réaction sur un composé de formule générale V

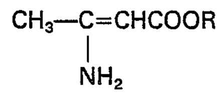

dans laquelle R est tel que défini dans la revendication 1 pour donner un composé de formule générale VI

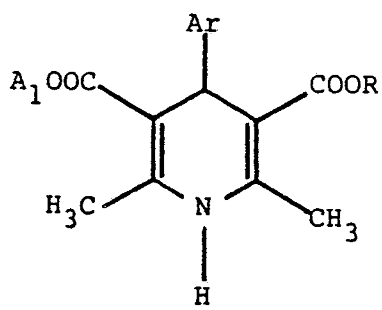

(VI)

dans laquelle $A_1$ est tel que défini dans cette revendication et Ar et R sont tels que définis dans la revendication 1, et, si $A_1$ représente un groupement de formule générale AX, en le convertissant en un groupement de formule générale IV.

4. Un procédé selon la revendication 3 dans lequel $A_1$ représente un groupement de formule AX et la conversion du groupement AX en un groupement de formule générale IV est effectuée en faisant réagir le composé de formule générale VI sur le dérivé de diphényl-3,3 propylamine de formule générale VII

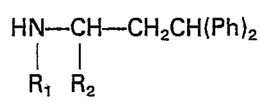

dans laquelle $R_1$, $R_2$ et Ph sont tels que définis dans la revendication 1.

5. Un procédé pour la préparation d'un diester selon la revendication 1, ledit procédé consistant à condenser un dérivé de la pyridine de formule générale

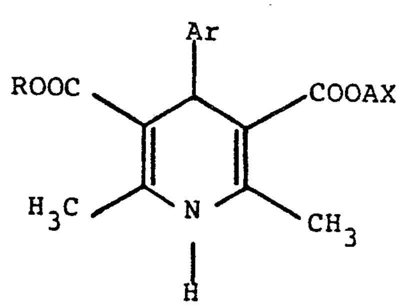

dans laquelle Ar, R et A sont tels que définis dans la revendication 1 et X représente un atome d'halogène, sur le dérivé de diphényl-3,3 propylamine de formule générale VII tel que défini dans la revendication 4.

6. Un procédé pour la préparation d'un diester selon la revendication 1, ledit procédé consistant à condenser un dérivé de la pyridine de formule générale

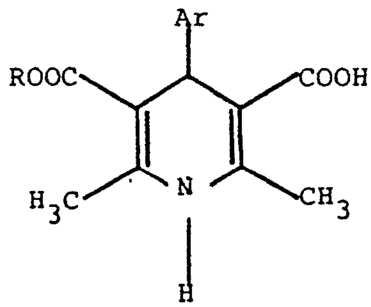

dans laquelle R et Ar sont tels que définis dans la revendication 1 sur un composé de formule générale VIII

$$HO{-}A{-}\underset{R_1}{\underset{|}{N}}{-}\underset{R_2}{\underset{|}{CH}}{-}CH_2CH(Ph)_2 \qquad (VIII)$$

dans laquelle A, $R_1$, $R_2$ et Ph sont tels que définis dans la revendication 1.

7. Un procédé pour la préparation d'un diester selon la revendication 1, ledit procédé consistant à condenser un dérivé de la pyridine de formule générale

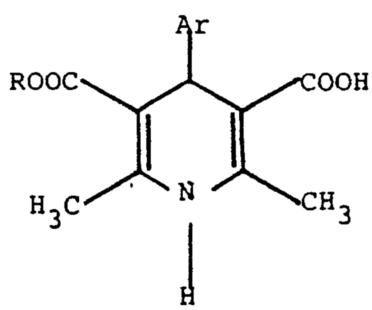

dans laquelle R et Ar sont tels que définis dans la revendication 1, sur un composé de formule générale YAX dans laquelle A est tel que défini dans la revendication 1 et X et Y représentent des atomes d'halogène, et en procédant selon la revendication 5.

8. Une composition pharmaceutique comprenant un diester selon la revendication 1 ou la revendication 2 ou un sel d'addition acide pharmaceutiquement acceptable de ceux-ci, mélangé avec un diluant ou un excipient pharmaceutiquement acceptable.

**Revendications pour l'Etat contractant: AT**

1. Un procédé pour la préparation d'un diester asymétrique de l'acide dihydro-1,4 diméthyl-2,6 pyridine dicarboxylique-3,5, l'ester répondant à la formule générale I

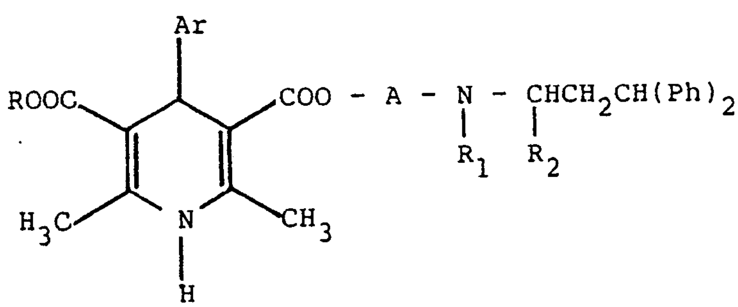

(I)

dans laquelle

Ph représente un groupement phényl,

Ar représente un groupement nitro-2 phényl, nitro-3 phényl, dichloro-2,3 phényl ou benzofurazane 4-yl,

A représente un groupement alcoylène en chaîne ramifiée comportant de 2 à 6 atomes de carbone,

R représente un groupement alcoyle en chaîne droite ou ramifiée ayant de 1 à 6 atomes de carbone, éventuellement monosubstitué par un groupement alcoxy ayant de 1 à 6 atomes de carbone,

$R_1$ représente un atome d'hydrogène, un groupement hydroxy ou un groupement alcoyle ayant de 1 à 4 atomes de carbone, et

$R_2$ représente un atome d'hydrogène ou un groupement méthyl;

ledit procédé consistant à condenser un composé de formule générale II

$$Ar{-}CHO \qquad (II)$$

dans laquelle Ar est tel que défini dans cette revendication, sur un composé de formule générale III

$$CH_3COCH_2COOA_1 \qquad (III)$$

dans laquelle $A_1$ représente (a) un groupement de formule générale IV

$$-A-\underset{R_1}{\underset{|}{N}}-\underset{R_2}{\underset{|}{CH}}-CH_2-CH(Ph)_2 \qquad (IV)$$

dans laquelle A, $R_1$, $R_2$ et Ph sont tels que définis dans cette revendication ou (b) un groupement de formule générale AX dans laquelle A est tel que défini dans cette revendication et X représente un atome d'halogène, à faire réagir le produit de la réaction sur un composé de formule générale V

$$CH_3-\underset{NH_2}{\underset{|}{C}}=CHCOOR$$

dans laquelle R est tel que défini dans cette revendication pour donner un composé de formule générale VI

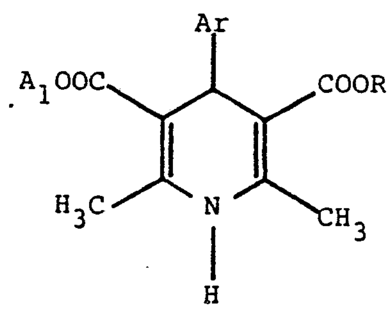

(VI)

dans laquelle $A_1$, Ar et R sont tels que définis dans cette revendication et, si $A_1$ représente un groupe de formule générale AX, en le convertissant en un groupement de formule générale IV.

2. Un procédé selon la revendication 1 dans laquel $A_1$ représente un groupement de formule AX et la conversion du groupement AX en un groupement de formule générale IV est effectuée en faisant réagir le composé de formule générale VI sur le dérivé de diphényl-3,3 propylamine de formule générale VII

$$H\underset{R_1}{\underset{|}{N}}-\underset{R_2}{\underset{|}{CH}}-CH_2CH(Ph)_2$$

dans laquelle $R_1$, $R_2$ et Ph sont tels que définis dans la revendication 1.

3. Un procédé pour la préparation d'un diester selon la revendication 1, ledit procédé consistant à condenser un dérivé de la pyridine de formule générale

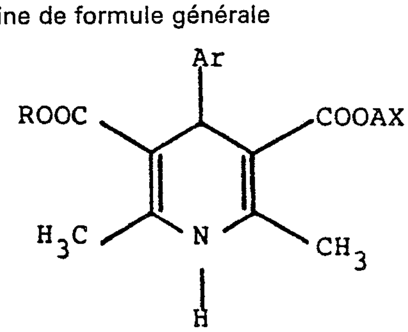

dans laquelle Ar, R, A et X sont tels que définis dans la revendication 1, sur le dérivé de diphényl-3,3 propylamine de formule générale VII tel que défini dans la revendication 2.

4. Un procédé pour la préparation d'un diester selon la revendication 1, ledit procédé consistant à condenser un dérivé de la pyridine de formule générale

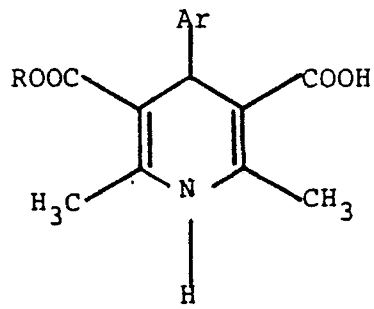

dans laquelle R et Ar sont tels que définis dans la revendication 1 sur un composé de formule générale VIII

$$\begin{array}{l} HO{-}A{-}\underset{\displaystyle R_1}{\underset{|}{N}}{-}\underset{\displaystyle R_2}{\underset{|}{CH}}{-}CH_2CH(Ph)_2 \end{array} \qquad (VIII)$$

dans laquelle A, $R_1$, $R_2$ et Ph sont tels que définis dans la revendication 1.

5. Un procédé pour la préparation d'un diester selon la revendication 1, ledit procédé ledit procédé consistant à condenser un dérivé de la pyridine de formule générale

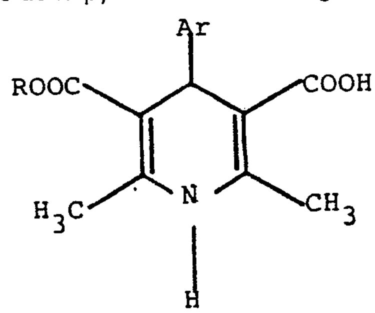

dans laquelle R et Ar sont tels que définis dans la revendication 1, sur un composé de formule générale YAX dans laquelle A est tel que défini dans la revendication 1 et X et Y représent des atomes d'halogène, et en procédant selon la revendication 3.